# EUROPEAN PATENT APPLICATION

(11) **EP 3 162 763 A1**
(43) Date of publication of application: **03.05.2017**
(21) Application number: 15290280.5
(22) Date of filing: 30.10.2015
(51) Int. Cl.: C01B 33/32, C07C 1/24, C07C 11/08, C07C 11/09

(54) **SIMULTANEOUS DEHYDRATION AND SKELETAL ISOMERISATION OF ISOBUTANOL ON TI-CONTAINING ZEOLITE CATALYSTS**

(71) Applicant: Total Research & Technology Feluy SA, 7181 Seneffe (Feluy) (BE); IFP Énergies nouvelles, 92852 Rueil-Malmaison Cedex (FR)
(72) Inventor: Danilina, Nadiya, 04315 Leipzig (DE); Nesterenko, Nikolai, 1402 Nivelles (Thines) (BE); Adam, Cindy, 5100 Wierde (BE); Minoux, Delphine, B-1400 Nivelles (BE); Vermeiren, Walter, 3530 Houthalen (BE); Dath, Jean-Pierre, 7970 Beloeil (BE); Coupard, Vincent, 69100 Villeurbanne (FR); Maury, Sylvie, 69440 Saint Maurice d'Argoire (FR)
(74) Representative: Mazurelle, Jean

(57) **Abstract**

The present invention (in a first embodiment) relates to a process for the simultaneous dehydration and skeletal isomerisation of isobutanol to make substantially corresponding olefins, having the same number of carbons and consisting essentially of a mixture of n-butenes and iso-butene, said process comprising:
a) introducing in at least one reactor a feed (A) comprising isobutanol, optionally water, optionally an inert component,
b) contacting said feed (A) with a catalyst in said at least one reactor at conditions effective to dehydrate and skeletal isomerise at least a portion of the isobutanol to make a mixture of n-butenes and iso-butene,
c) recovering from said at least one reactor an effluent (B) and removing water, the inert component if any and unconverted isobutanol if any to get a mixture of n-butenes and iso-butene ,

Wherein,
the catalyst comprises a Ti-containing zeolite, in which Ti atoms occupy framework positions of the zeolite, said catalyst being capable to make simultaneously the dehydration and skeletal isomerization of butene.

## Description

### [Field of the invention]

The present invention relates to the simultaneous dehydration and skeletal isomerisation of isobutanol to make a corresponding olefin, having substantially the same number of carbons but different skeleton structure. The limited supply and increasing cost of crude oil has prompted the search for alternative processes for producing hydrocarbon products such as isobutene and n-butenes. Isobutanol can be obtained by fermentation of carbohydrates or by condensation of lighter alcohols, obtained by fermentation of carbohydrates. Made up of organic matter from living organisms, biomass is the world's leading renewable energy source.

### [Background of the invention]

Historically Isobutanol (2-methyl-1-propanol, iBuOH) was produced as a by-product of Oxo Process from propylene (main product n-butanol) and has found limited applications. Its use resembles that of 1-butanol. It has been used as solvent, diluents, wetting agent, cleaner additive and as additive for inks and polymers.

Recently, a significant progress has been achieved in production of iBuOH via fermentation. The well established process to produce bio-ethanol from both 1^{st} & 2^{nd} generation of biomass can be retrofitted to the bio-iBuOH production facilities. GEVO and Butamax currently are the forerunners in bio-based iBuOH technologies. In spite of its established reputation, the Oxo Process does not appear to compete well anymore with leading bio-based routes. This opens the opportunities for new applications for bio-iBuOH feedstock. For example, isobutanol has already gained interest as fuel or fuel component as it exhibits a high octane number (Blend Octane R+M/2 is 102-103) and a low vapor pressure (RVP is 3.8-5.2 psi).

Literature reports have shown that the production costs of bio-iBuOH are potentially even lower than the costs to make n-BuOH. So, the bio-iBuOH feedstock is also attractive for chemical market, for example as pre-cursors for n-butenes or drop-in bio- Raffinate-1.

In addition iBuOH may also be produced from biomass via the base-catalysed Guerbet condensation of methanol with ethanol. Due to low costs and high availability of bio-ethanol globally, the Guerbert process has also a good chance for a widespread development, in particular in Asia region. Thus, dehydration of isobutanol obtained from biomass to corresponding olefins brings a perspective route to produce renewable feedstock for petrochemicals applications. Unfortunately, while direct conversion of isobutanol over a conventional dehydration catalyst leads to a product rich in isobutene, mostly linear butenes are interesting for chemical market, for example as feedstock for metathesis to produce propylene, for oxidative dehydrogenation to make butadiene or foruse as a co-polymer or for integration into a Raffinate I-pool.

The dehydration reactions of alcohols to produce alkenes have been known for a long time and many available solid acid catalysts can be used for alcohol dehydration. However, y-alumina is the most commonly used, especially for the longer chain alcohols (with more than three carbon atoms). This is because catalysts with stronger acidity, such as the silica-aluminas, molecular sieves, zeolites or resin catalysts can promote double-bond shift, skeletal isomerization and other olefin inter-conversion reactions. The primary product of the acid-catalysed dehydration of isobutanol over the y-alumina is iso-butene:

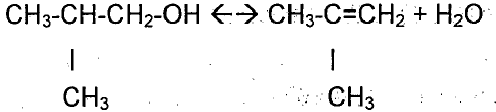

The dehydration of alcohols with four or more carbons over solid acid catalysts is expected to be accompanied by the double-bond shift reaction of the alkene product. This is because the two reactions occur readily and at comparable rates. The primary product, iso-butene is very reactive in presence of acid catalyst because of the presence of a double bond linked to a tertiary carbon. This allows easy protonation, as the tertiary structure of the resulting carbocation is the most favourable one among the possible carbocation structures (tertiary > secondary > primary carbocations). The resulting t-butyl-cation undergoes easy oligo/polymerisation or other electrophilic substitution on aromatics or aliphatics or electrophilic addition reactions. The rearrangement of t-butyl-cation is not a straightforward reaction as, without willing to be bound to any theory, it involves an intermediate formation of secondary or primary butyl-cation and hence the probability of secondary reactions (substitutions or additions) is very high and would reduce the selectivity for the desired product.

The skeletal isomerization of linear olefins to branched olefins is also a well-known process that can be realized over a zeolitic catalyst treated to have a moderate acidity. For example, the skeletal isomerization of n-butenes into isobutene over a Ferrierite catalyst is well known [US6,111,160, and US6,323,384]. Hence, many skeletal isomerisation catalysts for the conversion of n-butenes into isobutene have been developed, but the reverse skeletal isomerisation of isobutene into n-butenes has been rarely mentioned.

The international patent application WO2011/113834 describes a process for the simultaneous dehydration/isomerisation of isobutanol which results in a mixture of n-butenes (but-1-ene and but-2-ene) and iso-butene. The process uses particular catalysts chosen amongst crystalline silicates, dealuminated crystalline silicate, phosphorus modified crystalline silicate, all of the group FER, MWW, EUO, MFS, ZSM-48, MTT or TON having Si/Al higher than 10, or chosen among molecular sieves of the type silicoaluminophosphate of the group AEL, or silicated, zirconated or titanated alumina's or fluorinated alumina. Although selectivity to linear butenes is good, for example in presence of an H-Ferrierite catalyst, several percent of secondary products, such as heavies, are formed. The titanated alumina catalyst described is an alumina on the surface of which titanium is added. In other words; the titanium does not occupy a framework site of the zeolite structure.

There is therefore a need for a dehydration/isomerisation process of isobutanol with a higher selectivity to linear butenes combined with lower amount of secondary products, such as heavies.

It has now been discovered that the dehydration and the skeletal isomerisation of isobutanol can be carried out simultaneously using a Ti-containing zeolite catalyst in which Ti atoms are in tetrahedral position with a higher selectivity to linear butenes and a lower amount of secondary products. Ti-containing silicates, and preferably of the MFI or MEL group, appear particularly efficient.

Up to now, Ti-containing zeolite catalysts have not been reported for dehydration of alcohol or for reverse skeletal isomerization of iso-butenes.

Traditionally, zeolites are defined as crystalline micro porous aluminosilicates, comprising basic units having a tetrahedral structure formed by tetrahedral T-atoms (T = Si or Al) connected by O atoms. The arrangement of these atoms creates crystals with regular microporosity and well defined pores and cavities.

The presence of aluminum atoms in tetrahedral coordination in the zeolitic framework creates negative charges, which can be balanced by organic or inorganic cations (such as alkali or alkaline-earth cations), as well as by protons. The large ionic exchange capacity and the possibility to generate Brönsted acidity, offer them unique properties for cation exchange, absorption, separation, or heterogeneous acid catalysis.

Zeolitic structures having chemical compositions other than aluminosilicates can also be synthesized and heteroatoms such as B, Ge, Fe, V, Sn, Ti, Ga, among others, have been introduced in zeolite frameworks during the last thirty years. The presence of heteroatoms other than Al in the zeolite framework permits to introduce other chemical properties to the zeolite, offering new opportunities for these microporous molecular sieves. Among the different hetero atoms introduced in framework positions, Ti has been the subject of many studies, as revealed by the large number of synthesized titanosilicates.

The isomorphic substitution of Si and/or Al by Ti(IV) species in the zeolitic framework does not introduce negative charges, as it occurs with aluminosilicates, and consequently, their potential applicability as Brönstedacid acid materials can be neglected. However, Ti can act as a Lewis acid which together with the hydrophobic character of silicates, can catalyze diverse oxidation reactions of hydrocarbons using hydrogen peroxide as oxidizing agent.

In particular, the titanosilicate form of MFI (TS-1, US 4 410 501) and titanium-silicalite form of MEL (TS-2, Appl Catal, 1990, 58: L1) are known to possess a bi-directional medium pore system (openings close to 5.5 Å). This material has been applied as catalyst in different chemical processes on industrial scale, such as phenol hydroxylation, cyclohexanone ammoximation or propylene epoxidation. However, the literature fails to describe the application of these materials for butanol dehydration.

Without wishing to be bound by any theory, the diffusion restrictions through the medium pores of TS-1 & TS-2 zeolite vs the classic ZSM-5/ZSM-11 in combination with the absence of strong Bronsted acidity may lead to significantly higher selectivity to n-butenes

In the present specification, substances other than aluminosilicates but having the same structure as aluminosilicates are designed as "zeolite-type" or "zeolite-like" or "zeolitic" materials.

In the present specification, the term "zeolite" may include not only aluminosilicates but also substances (such as titanosilicates) having a structure analogous to aluminosilicates.

In the present specification, atoms in the framework position of a zeolite structure occupy the position of a Si or Al atom within the framework. In other words, they usually occupy a tetrahedral position within the framework. Atoms within the framework usually present a tetrahedral coordination. Such atoms in the framework position cannot be assimilated to atoms deposited on the surface of the zeolite.

By way of example, it has been discovered that for the simultaneous dehydration and skeletal isomerisation of isobutanol, Ti-containing crystalline silicates, in particular of the group MFI or MEL, have many advantages. N-butenes can be made beside iso-butene, the isobutanol conversion being at least 75% and often substantially complete (>99%). Advantageously, the n-butenes selectivity in the C4= fraction is above 50% %.

The isobutanol conversion is the ratio (isobutanol introduced in the reactor - isobutanol leaving the reactor)/ (isobutanol introduced in the reactor).

The n-butenes yield is the ratio, on carbon basis, (n-butenes leaving the reactor)/ (isobutanol introduced in the reactor).

The n-butenes selectivity is the ratio, on carbon basis, (n-butenes leaving, the reactor)/ (isobutanol converted in the reactor).

The n-butenes selectivity in the C4= fraction is the ratio, on carbon basis, (n-butenes leaving the reactor)/ (butenes leaving the reactor).

The simultaneous dehydration/isomerisation of isobutanol results in a mixture of n-butenes (but-1-ene and but-2-ene) and iso-butene.

A composition with n-butenes content rich in i-butene and significantly below thermodynamic equilibrium is often obtained on many alumina and zeolites while maintaining a high yield of total butenes. At 350°C the equilibrium i-butene content in butenes is approximately 50 mol%, at lower temperature the content of i-butene is even higher (Top Catal (2010) 53:1224-1230).

This contribution discloses a catalyst composition, which leads to a butene cut, which is rich in n-butenes and advantageously contains above 50wt% of n-butenes. An important advantage of the present invention is that the composition resembles the composition of a raffinate I C4 cut obtained from a steam naphtha cracker. Raffinate I is obtained by removing butadiene from the raw C4 cut produced on a steam naphtha cracker. Typical compositions are: 35-45% isobutene, 3-15% butanes and the remaining 52-40% n-butenes. It becomes evident that the product from the simultaneous dehydration/isomerisation can readily replace the use of raffinate I in existing petrochemical plants. The result is that capital investment can be minimised and that the derivatives from such iso-butene/n-butenes mixture can hence be produced from renewable resources instead of fossil resources simply by substituting fossil raffinate I by the product of the present invention.

### [Brief summary of the invention]

The present invention relates to a process for the simultaneous dehydration and skeletal isomerisation of isobutanol to make substantially corresponding olefins, having the same number of carbons and consisting essentially of a mixture of n-butenes and iso-butene, said process comprising:
a) introducing in a reactor a feed (A) comprising isobutanol, optionally water, optionally an inert component,
b) contacting said feed (A) with a catalyst in said reactor at conditions effective to dehydrate and skeletal isomerise at least a portion of the isobutanol to make a mixture of n-butenes and iso-butene,
c) recovering from said reactor an effluent (B) and removing water, the inert component if any and unconverted isobutanol if any to get a mixture of n-butenes and iso-butene,

Wherein,
the catalyst comprises a Ti-containing zeolite, in which Ti atoms occupy framework positions of the zeolite, said catalyst being capable to make simultaneously the dehydration and skeletal isomerization of butene.

In an embodiment, the Ti-containing zeolite is a zeolitic titanosilicate, preferably a crystalline titanosilicate.

In an embodiment, the zeolitic titanosilicate is of the group Ti-MFI (TS-1), Ti-MEL (TS-2), Ti-Beta, Ti-ZSM-12, Ti-ZSM-48, Ti-MOR, Ti-SSZ-33, T'i-MWW, Ti-FER (ECNU-8 obtained from PLS-3 layered precursor), TiITQ-7 or MCM-56.

In a preferred embodiment, the zeolitic titanosilicate is of the group MFI or MEL.

By way of example, the catalyst may be a TS-1 zeolite and can be made for instance as described in US4410501. TS-1 zeolite is the titanosilicate form of zeolite of MFI group. So, TS-1 has an MFI structure formed by a three-dimensional system of channels with 0.53 x 0.56nm and 0.51 x 0.51 nm. Ti(IV) atoms are incorporated into the framework in tetrahedral sites, substituting some Si atoms. TS-1 has a composition corresponding to the formula xTiO₂ (1-x)SiO₂, where x lies between 0.01 and 0.04. The TS-1 is of the silicalite type, where all the titanium substitutes the silicon.

The maximal amount of Ti may be limited by the structure of the Ti-containing zeolite. The minimum amount of Ti is sufficient to obtain selectively more n-butenes than isobutene. This can be determined by following the composition of the effluent.

By way of example, the zeolitic crystalline titanosilicate may have a Ti content below 3% by weight, advantageously of below 2% by weight, more advantageously of below 1.5% by weight and preferably of below 1% by weight and a minimum content of at least 0.5% wt, preferably at least 0.8%wt.

It would not depart from the scope of the invention if the isobutanol-comprising feed comprises one or more of the other C4 alcohols such as 2-butanol, tertiobutanol and n-butanol. Advantageously isobutanol is the major component among alcohols in the feedstock, this means the ratio of isobutanol to all the C4 alcohols in the feedstock is 42% or above. More advantageously the previous ratio is 70% or more and preferably 80% or more. Of course if the proportion of isobutanol is too low the invention is of low interest, there are a lot of catalysts in the prior art capable to dehydrate 2-butanol and n-butanol to produce n-butenes.

In an advantageous embodiment the effluent (B) is purified in a step d) to produce a n-butenes stream (N) and to remove the essential part of isobutene optionally recycled with feed (A) to the dehydration/isomerization reactor of step b). Recycling isobutene to the dehydration/isomerization reactor of step b) increases the n-butenes production.

In a specific embodiment, in the purification of step d) iso-butene is removed by selective oligomerisation (dimerization) of iso-butene.

In a specific embodiment in the purification of step d) iso-butene is removed by selective etherification with methanol or ethanol.

In a specific embodiment in the purification of step d) iso-butene is removed by selective hydratation into t-butanol. Optionally said t-butanol is recycled to the dehydration/isomerization reactor of step b).

In a specific embodiment, the process may comprise a further step in which the mixture of n-butenes and iso-butenes recovered from step c), from which isobutanol is optionally removed, is treated in at least one unit selected from a methathesis unit, an oligomerization unit or an oxidative dehydrogenation unit.

### [Detailed description of the invention]

**As regards the feed (A),** the isobutanol contained in the feed can be produced by any existing route.

Isobutanol may be produced from propylene via hydroformylation in the oxo-process (Rh-based catalyst) or via carbonylation in the Reppe-process (Co-based catalyst). Hydroformylation or carbonylation makes n-butanal and iso-butanal in ratios going from 92/8 to 75/25. To obtain isobutanol, the iso-butanal is hydrogenated over a metal catalyst.

Isobutanol can also be produced from synthesis gas (mixture of CO, H₂ and CO₂) by a process similar to Fischer-Tropsch, resulting in a mixture of higher alcohols, although often a preferential formation of isobutanol occurs.

Still another route to obtain isobutanol is the base-catalysed Guerbet condensation of methanol with ethanol and/or propanol.

Recently, new biochemical routes have been developed to produce selectively isobutanol from carbohydrates. The new strategy uses the highly active amino acid biosynthetic pathway of microorganisms and diverts its 2-keto acid intermediates for alcohol synthesis. 2-Keto acids are intermediates in amino acid biosynthesis pathways. These metabolizes can be converted to aldehydes by 2-keto-acid decacboxylases (KDCs) and then to alcohols by alcohol dehydrogenases (ADHs). Two non-native steps are required to produce alcohols by shunting intermediates from amino acid biosynthesis pathways to alcohol production (see for example US2008/0261230). Recombinant microorganisms are required to enhance the flux of carbon towards the synthesis of 2-keto-acids. In the valine biosynthesis 2-ketoisovalerate is an intermediate. Glycolyse of carbohydrates results in pyruvate that is converted into acetolactate by acetolactate synthase. 2,4-dihydroxyisovalerate is formed out of acetolactate, catalysed by isomeroreductase. A dehydratase converts the 2,4-dihydroxyisovalerate into 2-keto-isovalerate. In the next step, a keto acid decarboxylase makes isobutyraldehyde from 2-keto-isovalerate. The last step is the hydrogenation of isobutyraldehyde by dehydrogenase into isobutanol.

Preferably, the isobutanol comprised in feed (A) is obtained from biomass Of the described routes towards isobutanol above, the Guerbet condensation, the synthesis gas hydrogenation and the 2-keto acid pathway from carbohydrates are routes that can use biomass as primary feedstock. Gasification of biomass results in synthesis gas that can be converted into methanol or directly into isobutanol. Ethanol is already at very large scale produced by fermentation of carbohydrates or via direct fermentation of synthesis gas into ethanol. Methanol and ethanol resourced from biomass can be further condensed to isobutanol. The direct 2-keto acid pathway can produce isobutanol from carbohydrates that are isolated from biomass. Simple carbohydrates can be obtained from plants like sugar cane, sugar beet. More complex carbohydrates can be obtained from plants like maize, wheat and other grain bearing plants. Even more complex carbohydrates can be isolated from substantially any biomass, through unlocking of cellulose and hemicellulose from lignocelluloses.

In a specific embodiment, the isobutanol-comprising feed (A) contains below 1 wt ppm of nitrogen-containing compounds.

In a specific embodiment, the isobutanol-comprising feed (A) contains below 5 wt ppm of sulfur-containing compounds.

In a specific embodiment, the concentration of butanols in isobutanol-comprising feed (A) is at least 99wt%, preferably at least 99,5wt%. Advantageously, concentration of other organic compounds is below 0.5 wt%, carbon basis.

Isobutanol comprised in feed (A) may be subjected to simultaneous dehydration and skeletal isomerisation alone or in mixture with an inert medium.

The inert component is any component provided it is substantially not converted on the catalyst. Because the dehydration step is endothermic the inert component can be used as energy vector. The inert component allows reducing the partial pressure of the isobutanol and other reaction intermediates and will hence reduce secondary reactions like oligo/polymerisation. The inert component may be selected among water, nitrogen, hydrogen, CO₂ and saturated hydrocarbons. It may be such that some inert components are already present in the isobutanol because they were used or co-produced during the production of isobutanol. Examples of inert components that may already be present in the isobutanol are water and CO₂. The inert component may be selected among the saturated hydrocarbons having up to 10 carbon atoms, naphtenes. Advantageously it is a saturated hydrocarbon or a mixture of saturated hydrocarbons having from 3 to 7 carbon atoms, more advantageously having from 4 to 6 carbon atoms and is preferably pentane. An example of inert component can be any individual saturated compound, a synthetic mixture of the individual saturated compounds as well as some equilibrated refinery streams like straight naphtha, butanes etc. Advantageously the inert component is a saturated hydrocarbon having from 3 to 6 carbon atoms and is preferably pentane. The weight proportions of respectively isobutanol and inert component are, for example, 30-99.9/70-0.01 or 30-100/70-0 (the total being 100).

The feed (A) may be provided as a flowing stream, liquid or gaseous.

**As regards the at least one reactor,** it can be a fixed bed reactor (radial, isothermal, adiabatic, etc), a moving bed reactor or a fluidized bed reactor. A typical fluid bed reactor is one of the FCC type used for fluidized-bed catalytic cracking in the oil refinery. A typical moving bed reactor is of the continuous catalytic reforming type. The simultaneous dehydration/isomerisation may be performed continuously in a fixed bed reactor configuration using several reactors in series of equal or different sizes or a pair of parallel "swing" reactors. The various preferred catalysts of the present invention have been found to exhibit high stability This enables the dehydration/isomerisation process to be performed continuously in two parallel "swing" reactors wherein when one reactor is operating, the other reactor is undergoing catalyst regeneration. The catalyst of the present invention also can be regenerated several timers.

The simultaneous dehydration/isomerisation may be performed continuously in a moving bed reactor in which the catalyst circulates from a reaction zone to a regeneration zone and backwards with a residence time of the catalyst in the reaction zone of at least 12 hours. In each zone the catalyst behaves substantially like in a fixed bed reactor, but the catalyst moves slowly, by gravity or pneumatically through the respective zone. The use of a moving bed reaction allows accomplishing a continuous operation with no switching of the feedstock and regeneration gas from one reactor to another one. The reaction zone receives continuously the feedstock while the regeneration zone receives continuously the regeneration gas.

The simultaneous dehydration/isomerisation may be performed continuously in a fluidised bed reactor in which the catalyst circulates from a reaction zone to a regeneration zone and backwards with a residence time of the catalyst in the reaction zone of less than 12 hours. In each zone the catalyst is in a fluidised state and exhibit such a shape and size that it remains fluidised in the flow of the feedstock and reaction products or regeneration gas. The use of a fluidised bed reactor allows regenerating very rapidly deactivated catalyst by regeneration in the regeneration zone.

**As regards the pressure,** it can be any pressure but it is more easy and economical to operate at moderate pressure. By way of example the pressure of the reactor ranges from 0.5 to 30 bars absolute (50 kPa to 3 MPa), advantageously from 0.5 to 10 bars absolute (50 kPa to 1 MPa), more advantageously from 0.5 to 9 bars absolute (50 kPa to 0.9 MPa). Advantageously, the partial pressure of the isobutanol is lower than 5 bars absolute (0.5 MPa) and advantageously from 0.5 to 4 bars absolute (0.05 MPa to 0.4 MPa), preferably lower than 3.5 bars absolute (0.35 MPa) and more preferably lower than 2 bars absolute (0.2 MPa).

**As regards the temperature,** the temperature of the dehydration/isomerisation reactor may range from 200°C to 500°C, advantageously from 220°C to 500°C, more advantageously from 230°C to 450°C.

These reaction temperatures refer substantially to average catalyst bed temperature. The isobutanol dehydration is an endothermic reaction and requires the input of reaction heat in order to maintain catalyst activity sufficiently high and shift the dehydration thermodynamic equilibrium to sufficiently high conversion levels.

In case of fluidised bed reactors: (i) for stationary fluidised beds without catalyst circulation, the reaction temperature is substantially homogeneous throughout the catalyst bed; (ii) in case of circulating fluidised beds where catalyst circulates between a converting reaction section and a catalyst regeneration section, depending on the degree of catalyst backmixing the temperature in the catalyst bed approaches homogeneous conditions (a lot of backmixing) or approaches plug flow conditions (nearly no backmixing) and hence a decreasing temperature profile will install as the conversion proceeds.

In case of fixed bed or moving bed reactors, a decreasing temperature profile will install as the conversion of the isobutanol proceeds. In order to compensate for temperature drop and consequently decreasing catalyst activity or approach to thermodynamic equilibrium, reaction heat can be introduced by using several catalyst beds in series with interheating of the reactor effluent from the first bed to higher temperatures and introducing the heated effluent in a second catalyst bed, etc. When fixed bed reactors are used, a multi-tubular reactor can be used where the catalyst is loaded in small-diameter tubes that are installed in a reactor shell. At the shell side, a heating medium is introduced that provides the required reaction heat by heat-transfer through the wall of the reactor tubes to the catalyst.

**As** regards **the WHSV of the isobutanol-comprising feed, in particular the WHSV of the isobutanol,** it may ranges advantageously from 0.1 to 20 h⁻¹, preferably from 0.5 to 10 h⁻¹, more preferably from 1 to 9 h⁻¹.

**As regards the effluent** (**B**), it comprises essentially water, olefin, the inert component (if any) and unconverted isobutanol. Said unconverted isobutanol is supposed to be as less as possible. The olefin is recovered by usual fractionation means. Advantageously the inert component, if any, is recycled in the feed (A) as well as the unconverted isobutanol, if any. Unconverted isobutanol, if any, is recycled to the reactor in the feed (A).

Advantageously, the proportion of n-butenes in butene fraction is above 15wt%, advantageously above 40wt%, more advantageously above 45wt%, even more advantageously above 47wt%, preferably above 50%. The proportion of n-butenes in butene fraction may attain 60wt% or even 70wt%:

**As regards the catalyst,** it comprises a Ti-containing zeolite in which Ti atoms occupy framework positions of the zeolite.

In a specific embodiment, the catalyst may comprise a zeolitic titanosilicate, preferably a crystalline titanosilicate, of the group Ti-MFI (TS-1), Ti-MEL (TS-2), Ti-beta, Ti-ZSM-12, Ti-ZSM-48, Ti-MOR, Ti-SSZ-33, Ti-MWW, Ti-FER (ECNU-8 obtained from PLS-3 layered precursor), Ti-ITQ-7 or Ti-MCM-56. Preferably, the zeolitic titanosilicate is of MFI or MEL group.

Presence of other metal atoms within the framework of the zeolite is not excluded.

In a specific embodiment, titanosilicates particularly well-suited for use in the present invention may be prepared:
by direct synthesis, where Ti is introduced at the step of synthesis in form of organic or inorganic compounds,
by inserting Ti into an existing framework of a porous crystalline silicate, e.g., by contact with volatile titanium compounds as described in U.S. Patent No. 4,576,805,
by contact with a liquid phase source of titanium, e.g., (NH₄)₂ TiF₆ (aq), and TiF₄ (aq).

In a specific embodiment, the titanosilicate employed can be any micro - porous crystalline silicate material wherein titanium is substantially present in the framework, in other words in which the framework-titanium content is above or equal to 0.8% by weight. A description of such materials and their preparation can be found in the references cited below, incorporated therein by reference.

U.S. Patent No. 3,329,481 describes zeolites containing titanium in the framework position, which are prepared from siliceous materials and inorganic titanium compounds in the absence of organic bases. The empirical formula of the crystalline titanosilicate zeolite described is : (D_{2/*n*}O)*ₓ*:TiO₂:(SiO₂)*_{y}*, where D is a monovalent metal, a divalent metal, ammonium or hydrogen, *n* is the valence of D, *x* is a number from 0.5 to 3, and *y* is a number from about 1.0 to 3.5.

U.S. Patent No. 4,329,328 to McAnespie et al teaches a method of preparing titanosilicate material by mixing titanate solution with a source of silica.

In another specific embodiment, the Ti-containing zeolite may be a crystalline titanosilicate having a MFI structure (as for example TS-1). Such titanosilicate maybe prepared as explained in the following reference.

Patent No. 4,410,501 to Taramasso et al, incorporated therein by reference, discloses a titanium silicalite designated TS-1 and its use in a wide variety of organic conversion reactions including isomerization of n-paraffins and naphthenes, reforming, and polymerization of compounds containing olefin bonds. The material is prepared from a reaction mixture containing sources of silicon oxide, titanium oxide, alkaline oxide, nitrogenated organic base and water. The titanium oxide source may include hydrolyzable TiX₄, where X is selected from the group consisting of F, Cl, Br and I.

In another specific embodiment, the Ti-containing zeolite may be a titanium-silicalite (TS-2) having a silicalite-2 (MEL) structure (Appl Catal, 1990, 58: L1).

In another specific embodiment, the Ti-containing zeolite may be a titanium-silicalite Ti-beta (J Chem Soc, Chem Commun, 1992: 589), Ti-ZSM-12 (Zeolites, 1995, 15: 236), Ti-ZSM-48 (J Chem Soc, Chem Commun, 1992: 745), Ti-MOR (J Phys Chem, 1996, 100: 10316), Ti-SSZ-33 (Chem Commun, 2000: 761), Ti-MCM-22 (Ti-MWW) (Phys Chem B, 2001, 105: 2897), or MCM-56 (Chem. Commun, 2008: 6224).

In another specific embodiment, the Ti-containing zeolite may be a titanoborosilicate, for example prepared as disclosed in the following references. U.S. Patents Nos. 4,519,998 and 4,623,526, incorporated therein by reference, relate to a process for preparing crystalline titanoborosilicate by reacting titanium-containing compound and an alkali tetra hydroborate, sodium silicate, and alkylammonium cation. These two references also teach exchanging noble metals with the titanoborosilicate. Hydrogen forms of titanoborosilicate are taught as being prepared by calcining and ammonium-exchanging with ammonium chloride, ammonium nitrate and ammonium acetate.

In another specific embodiment, the Ti-containing zeolite may be a crystalline titanosilicate comprising other metal atoms in the framework positions, as the titanosilicates described in U.S. Patent No. 4,576,805 to Chang et al (incorporated therein by reference) which discloses a method for treating porous crystalline silicates by adding framework metals by contacting said silicates with volatile metal compounds, e.g., TiCl₄. The anhydrous anionic framework molar composition of the crystalline zeolite described is: (1-x) SiO₂:(*x*)MO_{*n*/2}, wherein x is less than or equal to 0.02, M is an initial lattice metal and n is the valence of M. M is selected from the metals of groups IIIB, IVB, VB, VIB, VIIB, VIII, IIIA, IVA, VA.

The crystalline titanosilicate can be subjected to various treatments before use in the present invention including, ion exchange, modification with metals (in a not restrictive manner alkali, alkali-earth, transition, or rare earth elements), external surface passivation, modification with P-compounds, steaming, acid treatment or other methods, or combination thereof.

In a specific embodiment the crystalline titanosilicate may contain aluminium atoms.

In a specific embodiment the titanosilicate is steamed before use in the reaction. The steam treatment is conducted at elevated temperature, preferably in the range of from 425 to 870°C, more preferably in the range of from 540 to 815°C and at atmospheric pressure and at a water partial pressure of from 13 to 200kPa. Preferably, the steam treatment is conducted in an atmosphere comprising from 5 to 100 vol% steam. The steam atmosphere preferably contains from 5 to 100 vol% steam with from 0 to 95 vol% of an inert gas, preferably nitrogen. The steam treatment is preferably carried out for a period of from 1 to 200 hours, more preferably from 4 hours to 10 hours. Such steam treatment tends to stabilize the catalyst before use in the iBuOH dehydration.

In another specific embodiment the crystalline Ti-containing zeolite of the invention or titanosilicate molecular sieve is mixed with a binder, preferably an inorganic binder, and shaped to a desired shape, *e.g.* pellets. In other words, the catalyst used in step b) of the invention comprises, or eventually consists of, a Ti-containing zeolite as described above and a binder. The binder is selected so as to be resistant to the temperature and other conditions employed in the dehydration process of the invention. The binder is an inorganic material, preferably a porous matrix material, and may be selected from clays, silica, metal silicates, metal oxides such as ZrO₂ and/or metals, or gels including mixtures of silica and metal oxides. By way of example, the binder may be selected from silica-alumina, silica-magnesia, silica-zirconia, silica-thoria, silica-beryllia, silica-titania, or ternary compositions such as silica=alumina-thoria, silica-alumina-zirconia, silica-alumina-magnesia and silica-magnesia-zirconia. If the binder which is used in conjunction with the crystalline zeolite is itself catalytically active, this may alter the conversion and/or the selectivity of the catalyst. In active materials for the binder may suitably serve as diluents to control the amount of conversion so that products can be obtained economically and orderly without employing other means for controlling the reaction rate. It is desirable to provide a catalyst having a good crush strength. This is because in commercial use, it is desirable to prevent the catalyst from breaking down into powder-like materials. Such clay or oxide binders have been employed normally only for the purpose of improving the crush strength of the catalyst. A particularly preferred binder for the catalyst of the present invention comprises silica. The relative proportions of the finely divided crystalline zeolite material and the inorganic oxide matrix of the binder can vary widely.

Typically, the binder content ranges from 1 to 90% by weight, advantageously from 5 to 95% by weight, more advantageously from 10 to 75% by weight, preferably from 10 to 50% by weight, based on the weight of the composite catalyst. Such a mixture of the crystalline zeolite and an inorganic oxide binder is referred to as a formulated crystalline zeolite. In mixing the catalyst with a binder, the catalyst may be formulated into pellets, extruded into other shapes, or formed into spheres or a spray-dried powder. Typically, the binder and the crystalline zeolite are mixed together by a mixing process. In such a process, the binder, for example silica, in the form of a gel is mixed with the crystalline zeolite material and the resultant mixture is extruded into the desired shape, for example cylindrical or multi-lobe bars. Spherical shapes can be made in rotating granulators or by oil-drop technique. Small spheres can further be made by spray-drying a catalyst-binder suspension. Thereafter, the formulated crystalline zeolite is calcined in air or an inert gas, typically at a temperature of from 200 to 900°C for a period of from 1 to 48 hours.

In addition, the mixing of the catalyst with the binder may be carried out either before or after any treatment steps.

**As regards the use of the product,** the mixture of n-butenes and iso-butene can replace the use of raffinate I in the refinery or petrochemical plants. The most typical application of such mixture is the conversion of the contained iso-butene into ethers (MTBE and ETBE), into t-butylalcohol (TBA) or oligomers (e.g. di/tri-iso-butenes), all being gasoline components. The higher oligomers of iso-butene can be used for jet fuel applications. High purity iso-butene can further be made by the decomposition of ethers (backcracking) or TBA (dehydration). High purity iso-butene finds applications in the production of Butyl-rubber, Poly-isobutene, Methylmethacrylate, Isoprene, Hydrocarbons resins, t-Butyl-amine, Alkyl-phenols and t-butyl-mercaptan.

Then-butenes, having not reacted during the production of ethers or TBA and substantially not or only to a limited extend during the oligomerisation, have applications in the production of sec-Butanol, Alkylate (addition of isobutane to butenes), Polygasoline, Oxo-alcohols and Propylene (metathesis with ethylene or self-metathesis between but-1-ene and but-2-ene). The n-butenes rich stream may be used for the production of butadiene via dehydrogenation or oxidative dehydrogenation.

The mixture of isobutene and butenes can be sent to a catalytic cracking which is selective towards light olefins in the effluent, the process comprising contacting said isobutene and butenes mixture with an appropriate catalyst to produce an effluent with an olefin content of lower molecular weight than that of the feedstock. Said cracking catalyst can be a silicalite (MFI or MEL type) or a P-ZSM5.

### [Examples]

### Example 1 (according the invention)

Catalyst A, according the invention, is a powder of TS-1 containing 1.67 wt% of Ti and 38.8wt% of Si prepared according to the method described in US4410501. Porous characteristics of catalyst A are gathered in table 1, showing that catalyst A is crystalline.

Catalyst A was sieved to 35-45 mesh (0.5 g) and activated in situ for 1 h at 500°C (2°C/min) in flow of He (50 ml/min).

**Table 1**

| | **Standard** | **Unit** | **Value** |
|---|---|---|---|
| BET Surface Area | | m²/g | 505 |
| External Surface Area (t-Plot): | ASTM D4365 | m²/g | 65.6 |
| Vtotal | ASTM D4365 | cm³/g | 0:288 |
| Vmicro (t-plot) | ASTM D4365 | cm³/g | 0.184 |

### Comparative example 2

Catalyst B is a silicalite zeolite H-ZSM-5 with a pure MFI structure having a Si/Al of 169 under powder form.

### Comparative example 3

Catalyst C is a γ-Al₂O₃ as 1.5mm extrudates which exhibits the following textural properties: a specific surface area of 285 m²/g, with a porous distribution centered around 94Å, and a porous volume of 0.67 ml/g. The impurities present on the alumina in small amounts are summarized below:
0.51 %wt S, 0.4%wt Si, 0.04%wt Ca, 0.08%wt Cl, 0.02%wt Fe, 0.01 %wt Cu.

### Comparative example 4

Catalyst D is a crystalline silicate of the FER structure. The H-FER has a Si/Al of 33 under powder form. The catalyst is calcinated with air at 550°C during 4 hours before formulation in pellets of 35-45 mesh.

### Example 5, catalytic tests at iso conversion

Catalysts A, B, C described above have been tested under the following operating conditions.

The catalytic tests are performed as follows: a flow of 50 ml/min of He is passed at atmospheric pressure through a saturator with isobutanol, which was maintained at 75°C, to entrain isobutanol into a reactor. That resulted in a WHSV of 4,7 h⁻¹(0.527 mmol/min or 2.34 g/h of iBuOH). The reaction temperature is 350°C.

The feed used is isobutanol (iBuOH) of 99% purity from Sigma-Aldrich (0,806 g/ml density at 15°C; 0,085 wt% H₂O).

Regeneration of the catalyst has been performed at following conditions: 10vol% O₂ in He (50 ml/min) at 550 °C (1°C/min) for 10 h.

Analysis of the products is performed by using an *on-line* gas chromatography. The lines between the saturator and the entrance to the *on-line* gas chromatography were heated to 150-160°C.

The results are presented in table 2 and can be summarized as follows.

Over Catalyst A (TS-1), at iso-conversion, less side products are formed, and the overall selectivity of butenes and 2-butenes is higher than for catalyst B (gamma-alumina).

**Table 2**

| Conversion, wt% | Selectivity wt % - C -basis | % | Selectivity, wt % - C -basis | Selectivity, wt % - C -basis | Selectivity, wt % - C -basis |
|---|---|---|---|---|---|
| | Butenes | nButenes/ Butenes | 1-butene | i-butene | 2-butenes |

| **Catalyst A (TS-1)** | | | | | |
|---|---|---|---|---|---|
| 100 | 99.0 | 54.4 | 7.2 | 45.1 | 46.7 |

| **Catalyst B (silicalite)** | | | | | |
|---|---|---|---|---|---|
| 100 | 80.8 | 47.8 | 5.6 | 43.2 | 33.0 |

| **Catalyst C (γ-Al₂O₃)** | | | | | |
|---|---|---|---|---|---|
| 100 | 99.9 | 8.5 | 0,0 | 91,6 | 8,4 |

### The data in the table are given on C-basis, coke free basis.

The data show that the TS-1 shows significantly higher selectivity to n-butenes relatively to a classic γ-Al₂O₃ dehydration catalyst.

The data also show that the TS-1 has a higher selectivity to n-butenes relatively to the zeolites of the same structure type but without titanium in the structure (silicalite, MFI). So, introducing Ti into zeolite is more advantageous versus a simple decreasing of zeolite activity by dealumination.

Finally, TS-1 shows very low side reaction activity relatively to the silicalite at high temperture. So; this zeolite allows a significantly wider temperature operating range for alcohol dehydration. This is important for a process design. As a reminder, dehydration of alcohol is often performed in a series of adiabatic reactors with an intermediate reheating and a big temperature difference between top and bottom of the catalytic bed (∼ 50-100 °C).

In this set of operating conditions, isobutanol conversion is almost complete with Catalyst A, with a butenes selectivity of about 99%wt, and an iso-butene selectivity of around 45%. Low amounts of by-products are formed.

### Example 6, catalytic tests at different temperatures

Catalysts A and D of examples 1 and 4 have been tested under the same conditions as example 5, but at two different temperatures: 350°C and 400°C.

The results are summarized in table 3 at the start of run (SOR) of the test.

The data demonstrate that the TS-1 catalyst is more selective to butenes and makes less by-producst than the H-Ferrierite (Catalyst D). In addition, in high temperature region, the selectivity on FER depends a lot on catalyst deactivation state, coke deposition and deactivation state. On the contrary, the TS-1 shows stable selectivity pattern. This means that the TS-1 allows better flexibility in operation and allows performing the dehydration of iBuOH at higher temperature (wider operating range).

This is again important for a process design. As a reminder, dehydration of alcohol is often performed in a series of adiabatic reactors with an intermediate reheating and a big temperature inlet and outlet difference in temperature (∼ 50-100 °C).

**Table 3**

| T,°C | Conversion (isobutanol) Wt% | Selectivities, wt %, C-basis | | | | | |
|---|---|---|---|---|---|---|---|
| | | ethylene | propylene | C5+ | Butenes | iso + 1-butene | 2-butene |
| **Catalyst A (TS1)** | | | | | | | |
| 350 | 100 | 0.00 | 0.55 | 0.40 | 99.0 | 52.3 | 46.7 |
| 400 | 100 | 0.00 | 0.59 | 0.32 | 99.0 | 58.1 | 40.9 |

| **Catalyst D (H-FER)** | | | | | | | |
|---|---|---|---|---|---|---|---|
| 350 | 100 | 0.52 | 12.4 | 10.5 | 74.3 | 44.3 | 30.0 |
| 400 | 100 | 1.28 | 9.0 | 5.4 | 83.1 | 48.9 | 34.2 |

**The data in the table are given on C-basis, coke free basis.**

## Claims

1. Process for the simultaneous dehydration and skeletal isomerisation of isobutanol to make substantially corresponding olefins, having the same number of carbons and consisting essentially of a mixture of n-butenes and iso-butene, said process comprising:
a) introducing in at least one reactor a feed (A) comprising isobutanol, optionally water, optionally an inert component,
b) contacting said feed (A) with a catalyst in said at least one reactor at conditions effective to dehydrate and skeletal isomerise at least a portion of the isobutanol to make a mixture of n-butenes and iso-butene,
c) recovering from said at least one reactor an effluent (B) and removing water, the inert component if any and unconverted isobutanol if any to get a mixture of n-butenes and iso-butene,
Wherein,
the catalyst comprises a Ti-containing zeolite, in which Ti atoms occupy framework positions of the zeolite, said catalyst being capable to make simultaneously the dehydration and skeletal isomerization of butene.

2. Process according to claim 1 wherein the Ti-containing zeolite is a zeolitic titanosilicate of the group MFI, MEL, Ti-beta, Ti-ZSM-12, Ti-ZSM-48, Ti-MOR, Ti-SSZ-33, Ti-MWW or MCM-56.

3. Process according to any one of the preceding claims wherein the WHSV of the isobutanol-comprising feed (A) is from 0.1 to 20 h⁻¹.

4. Process according to any one of the preceding claims wherein the pressure of the at least one reactor ranges from 0.5 to 30 bars absolute.

5. Process according to any one of the preceding claims wherein the temperature in the at least one reactor ranges from 200°C to 500°C.

6. Process according to any one of the preceding claims wherein the feed (A) provided in step a) comprises isobutanol and an inert component, the weight proportion of isobutanol to the sum of inert component and isobutanol being from 30 to-99.9 wt%.

7. Process according to any one of the preceding claims wherein the effluent (B) is purified in a step d) to produce a n-butenes stream (N) and to remove the essential part of isobutene optionally recycled with feed (A) to the dehydration/isomerization reactor of step b).

8. Process according to claim 7 wherein in the purification of step d) iso-butene is removed by one of the following operations :
- selective oligomerisation of iso-butene,
- selective etherification with methanol or ethanol,
- selective hydratation into t-butanol.

9. Process according to claim 8 wherein in the purification of step d) iso-butene is removed by selective hydratation into t-butanol and wherein said t-butanol is recycled to the dehydration/isomerization reactor of step b).

10. Process according to any one of the preceding claims wherein the isobutanol-comprising feed (A) contains below 1 wt ppm of nitrogen-containing compounds.

11. Process according to any one of the preceding claims wherein the isobutanol-comprising feed (A) contains below 5 wt ppm of sulfur-containing compounds.

12. Process according to any one of the preceding claims wherein the concentration of butanols in isobutanol-comprising feed (A) is at least 99wt%.

13. Process according to any one of the preceding claims, wherein among the butenes produced at step c) the proportion of n-butenes is above 40%.

14. Process according to claim 13 wherein among the butenes produced at step c) the proportion of n-butenes is above 50%.

15. Process according to any of the preceding claims comprising a further step in which the mixture of n-butenes and iso-butenes recovered from step c), from which isobutanol is optionally removed, is treated in at least one unit selected from a methathesis unit, an oligomerization unit or an oxidative dehydrogenation unit.
